(19) Europäisches Patentamt
European Patent Office
Office européen des brevets

(11) **EP 4 682 529 A1**

(12) **EUROPEAN PATENT APPLICATION**

(43) Date of publication:
**21.01.2026 Bulletin 2026/04**

(21) Application number: **25190320.9**

(22) Date of filing: **17.07.2025**

(51) International Patent Classification (IPC):
*G01N 27/403* (2006.01)   *G01N 27/416* (2006.01)
*G01N 33/483* (2006.01)   *G01N 33/487* (2006.01)
*C12M 1/34* (2006.01)

(52) Cooperative Patent Classification (CPC):
**G01N 27/403; G01N 27/416; G01N 33/4836;**
C12M 21/06; C12M 41/46; G01N 33/48728;
G01N 33/48735

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **18.07.2024 TW 113126926**

(71) Applicants:
• **Chung Shan Medical University**
**Taichung City 402 (TW)**
• **National Chung Hsing University**
**Taichung City 402 (TW)**

(72) Inventors:
• **LEE, MAW-SHENG**
**402306 Taichung City (TW)**

• **WU, CHING-CHOU**
**402202 Taichung City (TW)**
• **YANG, PING-FENG**
**402202 Taichung City (TW)**
• **LEE, CHIA-EN**
**402202 Taichung City (TW)**
• **CHENG, EN-HUI**
**402306 Taichung City (TW)**
• **LIN, YI-PING**
**402306 Taichung City (TW)**
• **TSAI, HAN-NI**
**402306 Taichung City (TW)**
• **LEE, TSUNG-HSIEN**
**402306 Taichung City (TW)**
• **LEE, CHUN-I**
**402306 Taichung City (TW)**

(74) Representative: **DTS Patent- und Rechtsanwälte PartmbB**
**Brienner Straße 1**
**80333 München (DE)**

(54) **ELECTROCHEMISTRY DETECTION CHIP**

(57)    An electrochemistry detection chip (10, 20) comprises: a base layer (11, 21), an electrode chip (12, 22), an insulating layer (13, 23), a reference material layer (14, 24), a first structural layer (15, 25) which is disposed on the insulating layer (13, 23) and comprises: a plurality of bottom support arms (151, 251), wherein the ends of the bottom support arms (151, 251) are adjacent to each other to jointly define a specimen accommodation area (152, 252); a covering layer (16, 26), and a salt bridge connecting layer (17, 27). The electrochemistry detection chip (10, 20) of the present invention can be used to detect the respiratory activity of embryos.

FIG. 1

EP 4 682 529 A1

**Description**

BACKGROUND OF THE INVENTION

1. Field of the Invention

The present disclosure relates to an electrochemistry detection chip, specially relates to an electrochemistry detection chip which comprises a reference material layer, a first structural layer and a covering layer.

2. Description of the Related Art

Traditionally, an oxygen consumption rate (OCR) of a single embryo or a spherical cell cluster can be measured by an electrochemistry detection chip based on the electrochemistry principle, and assesses the situation of the single embryo or the spherical cell cluster and the application value thereof.

For example, with the specific application of electrochemistry detection chips, the success rate of clinical pregnancy is only 50-60% by single embryo transfer, which means not every embryo can develop successfully. Although morphology assessment and advanced artificial intelligence image-analysis tools can help to partly identify a healthy embryo and raise the pregnancy rate to about 65%, these methods are subjective, time-consuming, and expensive. Furthermore, the embryo's mitochondrial activity is another biomarker that indicates the morula and blastocyst's healthy status. The Igenomix company uses the mitochondria's mtDNA to calculate MitoScore as a mitochondrial biomarker. However, the MitoScore does not provide direct evidence of the pregnancy rate. Thus, it is very important for infertility patients to assess the embryo's mitochondrial activity, which can be estimated by oxygen consumption rate (OCR) due to the oxidative phosphorylation reaction of mitochondria. The embryo OCR value can be detected using an electrochemistry detection chip, which is label-free and noninvasive.

JP2010121948A discloses a device for measuring the respiration activity of the fertilized egg, which integrates a platinum reference electrode and a working electrode on the same substrate, fixes an embryo on a single-thickness microchannel composed of $SiO_2$, and lets the embryo lower than a sensor electrode substrate. The working electrode can be single or multiple working electrodes.

US10458941B2 discloses an electrochemical measurement device, it integrates an upper accumulater with its conical structural layer, and the below electrochemical measurement device which contains the multiple ring working electrodes and a ring counter electrode on a same substrate, fixes an embryo on a single-thickness microwell composed of insulating substrate, and makes the detection pores exposure on the single ring working electrode by insulating substrate.

CN107003265A discloses an electrochemical measurement device, it integrates the multiple ring working electrodes and a ring counter electrode on a same substrate, fixes an embryo on a single-thickness microwell composed of $SiO_2$, wherein the depth of the microwell is designed to equal with or smaller than the embryo radius, to make it concaves toward the sensor electrode substrate. It makes multiple detection pores exposed on the ring single working electrode by an insulating substrate.

CN117030824A discloses a single cell respiration rate measuring sensor based on glass substrate, it integrates an upper accumulater with a six-conical structural layer and a below electrochemical measurement device which contains the multiple ring working electrodes, a Pt semi-circle reference electrode and a Pt semi-circle counter electrode on a same substrate, then fixes the embryo on the single-thickness microwell composed of insulating substrate which is made by dry etching. The concentric circle working electrode array is set by 5 microwells, and makes multiple detection pores exposure on the ring single working electrode by insulating substrate. In the non-working microwell, the Pt reference electrode and counter electrode are usually placed in the conical structural microwell closest to the edge.

TW201120441A1 discloses a dissolved-oxygen (DO) electrode array with the integration of cell-patterned culture used for estimation of cellular respiratory activity, consisting of a sensing chip and a microwell-levitated slab, wherein the sensing chip includes a substrate and an insulating layer placed on a substrate, and the substrate contains a cell-cultured area, a counter electrode, and multi-DO electrodes. Each DO electrode and the counter electrode have a working window. The insulator layer covers on the counter electrode and the multi-DO electrodes, and a cell-culture hole, which is used as a cell-adhesion region, is placed on the insulator. The distance between the center of the cell-adhesion region and the working window is smaller than the five times radius of cell-cultured area. The microwell-levitated layer is combined with the sensing chip.

However, the reference potential and stability of specimen fixing of the electrochemistry detection chips can be further optimized in prior arts. Therefore, it is necessary to provide a novel electrochemistry detection chip to meet the needs of the industry.

BRIEF SUMMARY OF THE INVENTION

As mentioned above, the electrochemistry detection chips can be further optimized in prior arts. Therefore, one purpose of the present disclosure is to raise the detection efficiency of an electrochemistry detection chip by comprising a reference material layer, a first structural layer, and a covering layer.

To achieve at least the above objective, the present disclosure provides an electrochemistry detection chip which comprises:

a base layer which is composed of impermeable material;

an electrode chip which is disposed on the base layer, and comprises at least a working electrode, a reference electrode, and a counter electrode;

an insulating layer which is covered on the electrode chip, and makes the working electrode exposed at the end only;

a reference material layer which is disposed on the reference electrode and is composed of silver chloride (AgCl);

a first structural layer which is disposed on the insulating layer and includes:

a plurality of bottom support arms, wherein the ends of the bottom support arms are adjacent to each other to jointly define a specimen accommodation area;

a covering layer which is disposed on the base layer and is composed of impermeable material, wherein the covering layer has a first pore and a second pore, wherein the first pore and a top surface of the base layer jointly define a working well, and an end of the working electrode and the counter electrode, and the specimen accommodation area are located in the working well; wherein the second pore and a top surface of the base layer jointly define a reference well, and an end of the reference electrode is located in the reference well;

a salt bridge connecting layer which is disposed on the base layer, and one end of it is located in the working well, and the other end of it is located in the reference well.

**In** an embodiment of the present disclosure, the electrochemistry detection chip can further comprises:
a second structural layer which is disposed on the first structural layer and comprises:
a plurality of upper support arms, wherein the upper support arms correspond to the settings of the bottom support arms and are adjacent to the specimen accommodation area, and the ends of the upper support arms and the bottom support arms form a stepped structure.

In an embodiment of the present disclosure, the salt bridge connecting layer is encircled by the insulating layer. This is because, in this embodiment, during the manufacturing process of the electrochemistry detection chip, the insulating layer is not only formed on the electrode chip to cover it, but also further surrounds the area that will be used to form the salt bridge connecting layer later. Therefore, when a liquid raw material (for example, a polyelectrolyte) is subsequently used to make salt bridge connecting layer, the insulating layer can be used to confine the liquid raw material to the area where the salt bridge connecting layer is to be formed. Thus, the liquid raw material can subsequently solidify to form a salt bridge connecting layer having a desire shape and position.

In an embodiment of the present disclosure, the reference material layer further contains Ag to enhance its chemical reaction equilibrium between AgCl and Ag.

In an embodiment of the present disclosure, the end of the upper support arms, which are adjacent to the specimen accommodation area, can define a specimen accommodation area with a diameter of 100-300 $\mu$m.

In an embodiment of the present disclosure, the first structural layer can further comprise:

a bottom frame, wherein the bottom support arms extend inward from the bottom frame;
wherein the second structural layer further comprises:
an upper frame corresponds to the setting of the bottom frame, wherein the upper support arms extend inward from the upper frame.

In an embodiment of the present disclosure, the electrode chip comprises a plurality of working electrodes, and the plurality of working electrodes have different distances from the center of the specimen accommodation area.

In an embodiment of the present disclosure, the electrode chip comprises 5 working electrodes.

In an embodiment of the present disclosure, the specimen accommodation area can be suitable for accommodating a single embryo or a spherical cell cluster.

The present disclosure provides an electrochemistry detection chip which can be a detection chip of an electrochemical dissolved oxygen (DO) array, and used to assess the oxygen consumption rate (OCR) of the embryo. The electrochemistry detection chip of the present disclosure meets the needs of clinical embryologists which can move the embryo to the chip manually and locate the distance between embryo location and electrode array. The electrochemistry detection chip of the present disclosure can precisely locate a single embryo to the center of DO array by the first structural layer around the DO array. A platinum (Pt) thin film electrode can use to become five working electrodes and a counter electrode in the electrochemistry detection chip. The electrochemistry detection chip uses a connecting Ag/AgCl reference electrode, and makes the connecting Ag/AgCl reference electrode locate in the independent reference well by the covering layer; it can effectively reduce the amplitude of reference potential fluctuations and significantly reduce the electrolyte leakage from the reference well to the working well.

In the electrochemistry detection chip of the prior arts, the working electrode and the reference electrode are usually located in the same well, or using the externally inserted reference electrode. When the working electrode and the reference electrode are located in the same well, if disposing the reference material layer which is composed of AgCl on the reference electrode, it will cause the undesirable influences to specimen physiological functions by AgCl toxicity. In contrast, the working electrode and the reference electrode are respectively located in the working well and the reference well in the electrochemistry detection chip of the present disclosure, these two wells are connected by the salt bridge connecting layer, and the fluid is disable to inflow from reference well to working well, it means these two wells are essentially independent of each other in fluids. Thereby, the electrochemistry detection chip of the present disclosure can raise the stability of the reference potential by disposing the reference material layer, which is composed of AgCl, on the reference electrode. And based on the reference electrode being located in the reference well, which is independent of the working well in fluids, the AgCl, which constitutes the reference material layer, will not be able to cause undesirable influences on the specimen.

The previous electrochemistry detection chip uses a single microwell with an inverted cone shaped continuous wall to drain the specimen (such as embryo) fall onto the detection site of the bottom, this design needs the precise operation under microscope to make the specimen locate on the suitable detection site on microwell, because the vortex will be produced by specimen injection. In contrast, the electrochemistry detection chip of the present disclosure uses the first structural layer which comprises a plurality of bottom support arms to drain the specimen into the suitable detection site, between the plurality of bottom support arms, it provides the space for fluid to discharge compared to the prior arts. It avoids the vortex which is produced by specimen injection, and can effectively reduce the operating difficulty and raise the stability of specimen fixing.

The electrochemistry detection chip of the present disclosure can effectively raise the reference potential and the stability of specimen fixing by comprising the reference material layer, the first structural layer and the covering layer, and improves the detection efficiency.

BRIEF DESCRIPTION OF THE DRAWINGS

For clarity, the thickness or dimensions of each layer in the figure have been enlarged, omitted or simplified. Additionally, the dimensions of each component do not necessarily reflect the actual size.

FIG. 1 is a top view of the electrochemistry detection chip of Example 1;
FIG. 2 is an enlarged presenting drawing of the working well of the electrochemistry detection chip of Example 1;
FIG. 3 is a further enlarged presenting drawing of the specimen accommodation area of the electrochemistry detection chip of Example 1;
FIG. 4 is a top view of the electrochemistry detection chip of Example 2;
FIG. 5 is an enlarged presenting drawing of the working well of the electrochemistry detection chip of Example 2;
FIG. 6 is a further enlarged presenting drawing of the specimen accommodation area of the electrochemistry detection chip of Example 2;
FIG. 7 is a test result of the reference potential of connecting Ag/AgCl reference electrode of the electrochemistry detection chip of Example 2;
FIG. 8 is a picture of the finished product of the electrochemistry detection chip of Example 1;
FIG. 9 is a micrograph of the mice embryo placed on the specimen accommodation area of the electrochemistry detection chip of Example 2;
FIG. 10 is a current measurement result of the Test Example; and
FIG. 11 is a OCR measurement result of different diameter embryo of Test Example.

DETAILED DESCRIPTION OF THE INVENTION

To facilitate understanding of the object, characteristics and effects of this present disclosure, it will now be described in detail by the following specific embodiments:

Example 1:

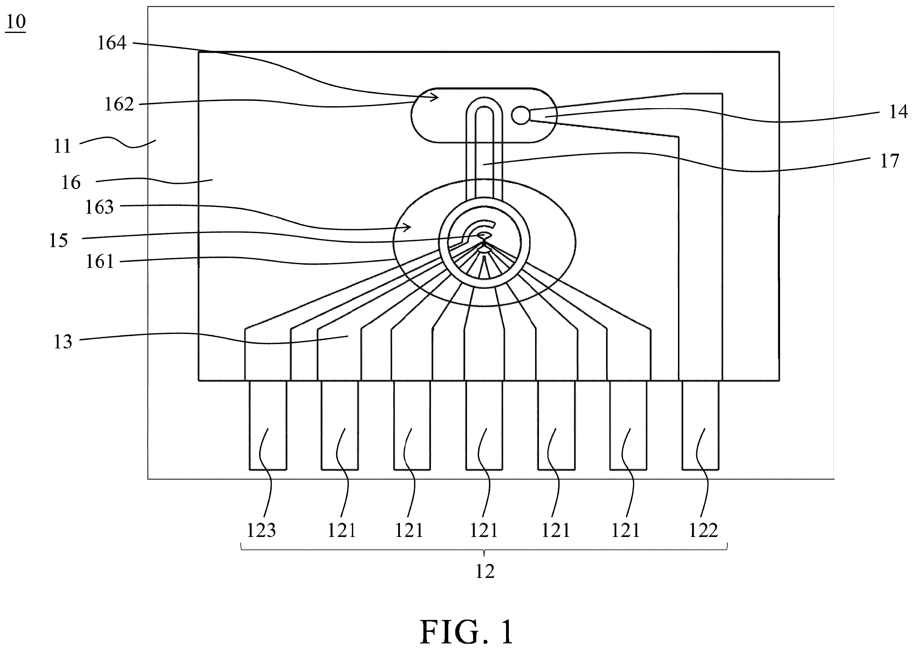

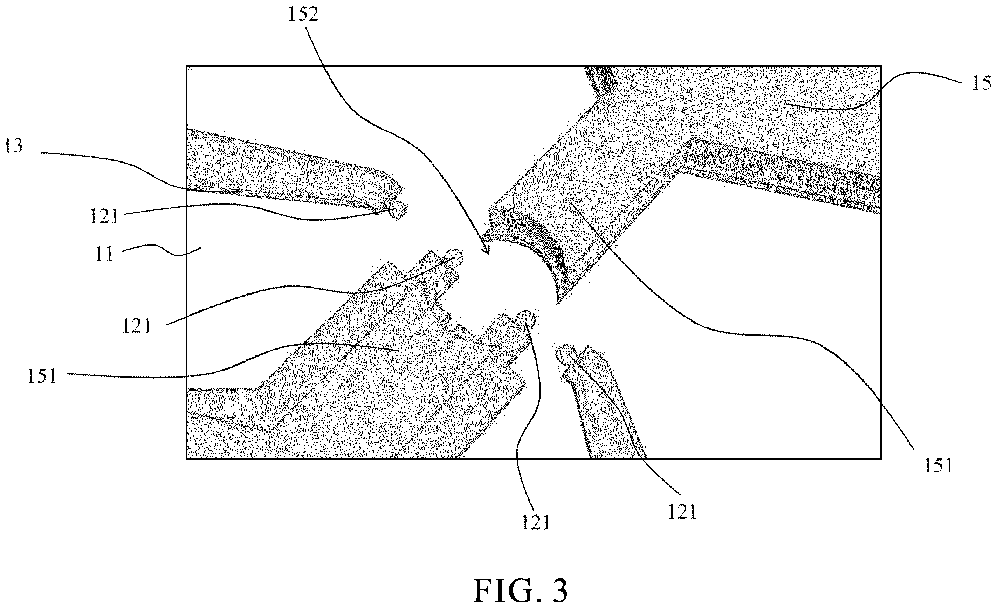

Referring to FIG. 1, 2 and 3, the electrochemistry detection chip 10 of Example 1 comprises: a base layer 11 which is composed of impermeable material. For example, the base layer 11 can be a slide, but the present disclosure is not limited thereto.

The electrochemistry detection chip 10 of Example 1 also comprises: an electrode chip 12 which is disposed on the base layer 11 and comprises at least a working electrode 121, a reference electrode 122 and a counter electrode 123. In Example 1, the electrode chip 12 comprises 5 working electrodes 121, but the present disclosure is not limited thereto.

The electrochemistry detection chip 10 of Example 1 also comprises: an insulating layer 13 which is covered on the electrode chip 12 and makes the working electrode 121 exposure the end only.

The electrochemistry detection chip 10 of Example 1 also comprises: a reference material layer 14 which is disposed on the reference electrode 122 and is composed of AgCl.

The electrochemistry detection chip 10 of Example 1 also comprises: a first structural layer 15 which is disposed on the insulating layer 13, comprises: a plurality of bottom support arms 151, wherein the ends of the bottom support arms 151 are adjacent to each other to jointly define a specimen accommodation area 152. In Example 1, the first structural layer 15 comprises 2 bottom support arms 151, but the present disclosure is not limited thereto.

The electrochemistry detection chip 10 of Example 1 also comprises: a covering layer 16 which is disposed on the base layer 11 and is composed of impermeable material, wherein the covering layer 16 has a first pore 161 and a second pore 162, wherein the first pore 161 and a top surface of the base layer 11 jointly define a working well 163, and an end of the working electrode 121 and the counter electrode 123, and the specimen accommodation area 152 are located in the working well 163; wherein the second pore 162 and a top surface of the base layer 11 jointly define a reference well 164, and an end of the reference electrode 122 is located in the reference well 164.

The electrochemistry detection chip 10 of Example 1 also comprises: a salt bridge connecting layer 17 which is disposed on the base layer 11 and an end of it is located in the working well 163, and the other end of it is located in the reference well 164. Wherein, the salt bridge connecting layer 17 is composed of a conducting polymer, in the preferred embodiment, the salt bridge connecting layer 17 is composed of a proton exchange membrane (PEM), but the present disclosure is not limited thereto.

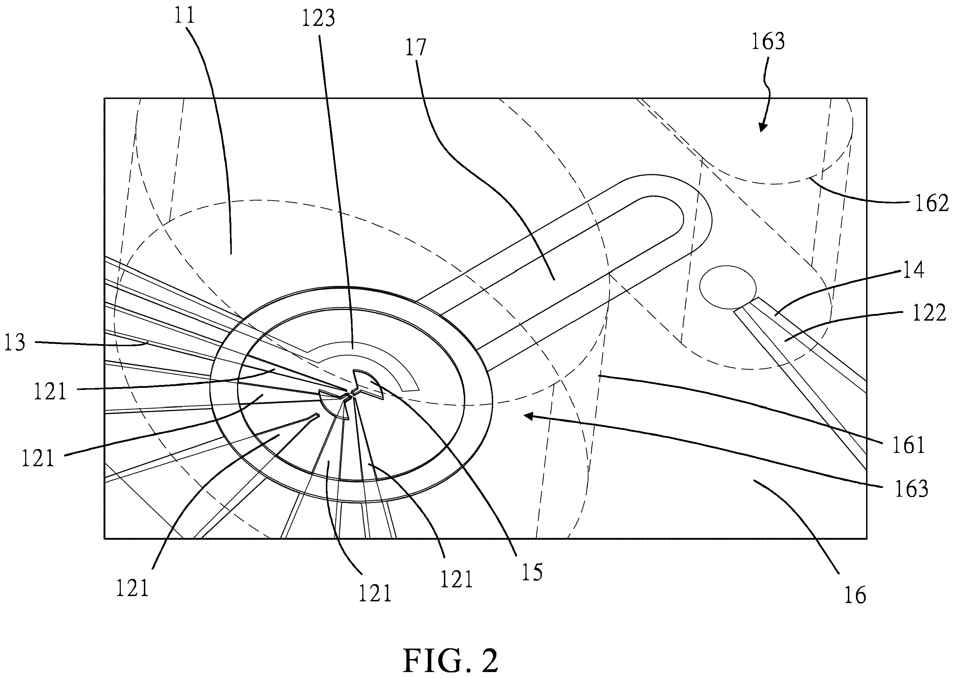

As shown in FIG. 1 and 2, the salt bridge connecting layer 17 is encircled by the insulating layer 13. This is because, in Example 1, during the manufacturing process of the electrochemistry detection chip 10, the insulating layer 13 is not only formed on the electrode chip 12 to cover it, but also further surrounds the area that will be used to form the salt bridge connecting layer 17. Therefore, when a polyelectrolyte is subsequently used to make the salt bridge connecting layer 17 to form a proton-exchange membrane, the insulating layer 13 can be used to confine the polyelectrolyte to the area where the salt bridge connecting layer 17 is to be formed. Thus, the polyelectrolyte can subsequently solidify to form a salt bridge connecting layer 17 having a desire shape and position. However, the present invention is not limited thereto.

Example 2:

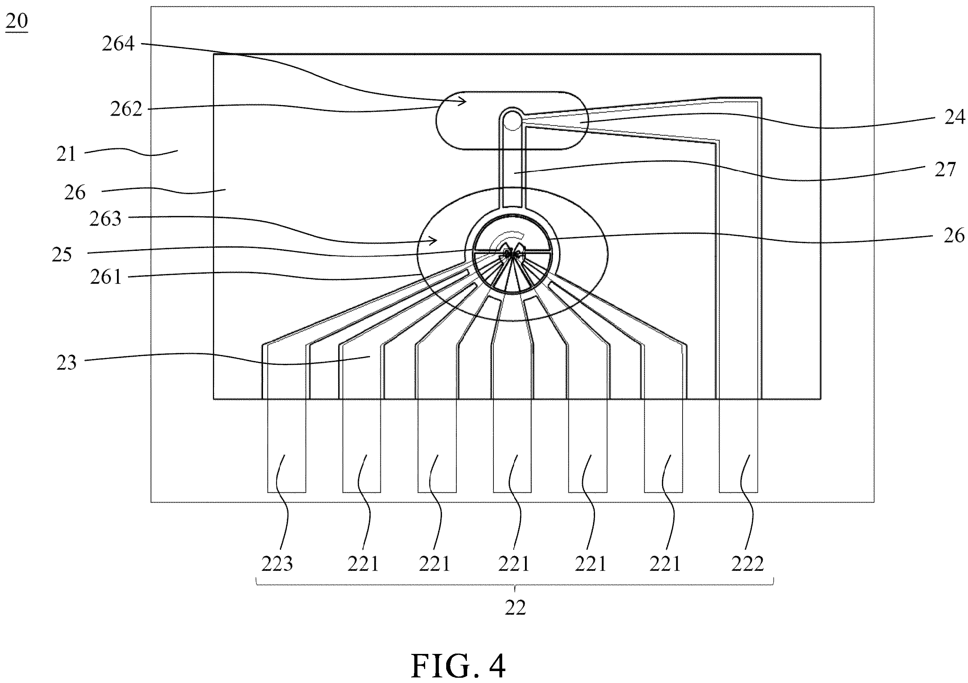

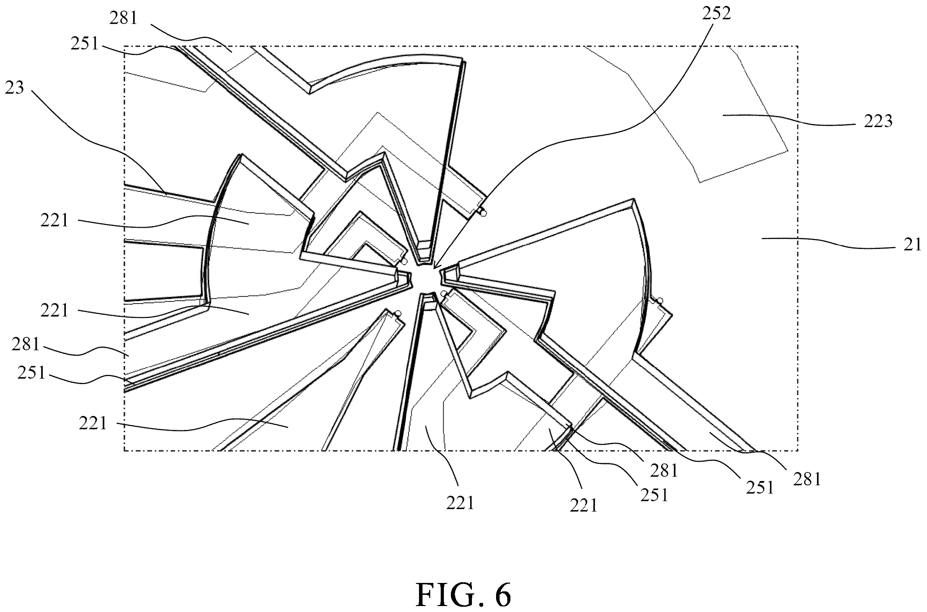

Referring to FIG. 4, 5 and 6, the electrochemistry detection chip 20 of Example 2 comprises: a base layer 21 which is composed of impermeable material. For example, the base layer 21 can be a slide, but the present disclosure is not limited thereto.

The electrochemistry detection chip 20 of Example 2 also comprises: an electrode chip 22 which is disposed on the base layer 21 and comprises at least a working electrode 221, a reference electrode 222 and a counter electrode 223. In Example 2, the electrode chip 22 comprises 5 working electrodes 221 and these working electrodes 221 have different distance with the center of the specimen accommodation area 252, but the present disclosure is not limited thereto.

The electrochemistry detection chip 20 of Example 2 also comprises: an insulating layer 23 which is covered on the electrode chip 22 and makes the working electrode 221 exposure the end only.

The electrochemistry detection chip 20 of Example 2 also comprises: a reference material layer 24 which is disposed on the reference electrode 222 and is composed of AgCl. In example 2, the reference material layer 24 further contains Ag to enhance its chemical reaction equilibrium between AgCl and Ag, but the present invention is not limited thereto.

The electrochemistry detection chip 20 of Example 2 also comprises: a first structural layer 25, which is disposed on the insulating layer 23, comprises: a plurality of bottom support arms 251, wherein the ends of the bottom support arms 251 are adjacent to each other to jointly define a specimen accommodation area 252. In Example 2, the first structural layer 25 comprises 4 bottom support arms 251, but the present disclosure is not limited thereto.

The electrochemistry detection chip 20 of Example 2 also comprises: a covering layer 26, which is disposed on the base layer 21, and is composed of impermeable material, wherein the covering layer 26 has a first pore 261 and a second pore 262, wherein the first pore 261 and a top surface of the base layer 21 jointly define a working well 263, and an end of the working electrode 221 and the counter electrode 223, and the specimen accommodation area 252 are located in the working well 263; wherein the second pore 262 and a top surface of the base layer 21 jointly define a reference well 264, and an end of the reference electrode 222 is located in the reference well 264.

The electrochemistry detection chip 20 of Example 2 also comprises: a salt bridge connecting layer 27, which is disposed on the base layer 21, and an end of it is located in the working well 263, and the other end of it is located in the reference well 264.

Compared to Example 1, the electrochemistry detection chip 20 of Example 2 also comprises: a second structural layer 28 which is disposed on the first structural layer 25 and comprises: a plurality of upper support arms 281, wherein the upper support arms 281 correspond to the settings of the bottom support arms 251 and are adjacent to the specimen accommodation area 252, and the ends of the upper support arms 281 and the bottom support arms 251 form a stepped structure.

Compared to Example 1, the first structural layer 25 of the electrochemistry detection chip 20 of Example 2 further comprises: a bottom frame 253, wherein the bottom support arms 251 extend inward from the bottom frame 253; wherein the second structural layer 28 further comprises: an upper frame 283 which corresponds to the setting of the bottom frame 253, wherein the upper support arms 281 extend inward from the upper frame 283. The electrochemistry detection chip of Example 2 can further strengthen the structures of the first structural layer 25 and the second structural layer 28 by disposition of the bottom frame 253 and upper frame 283.

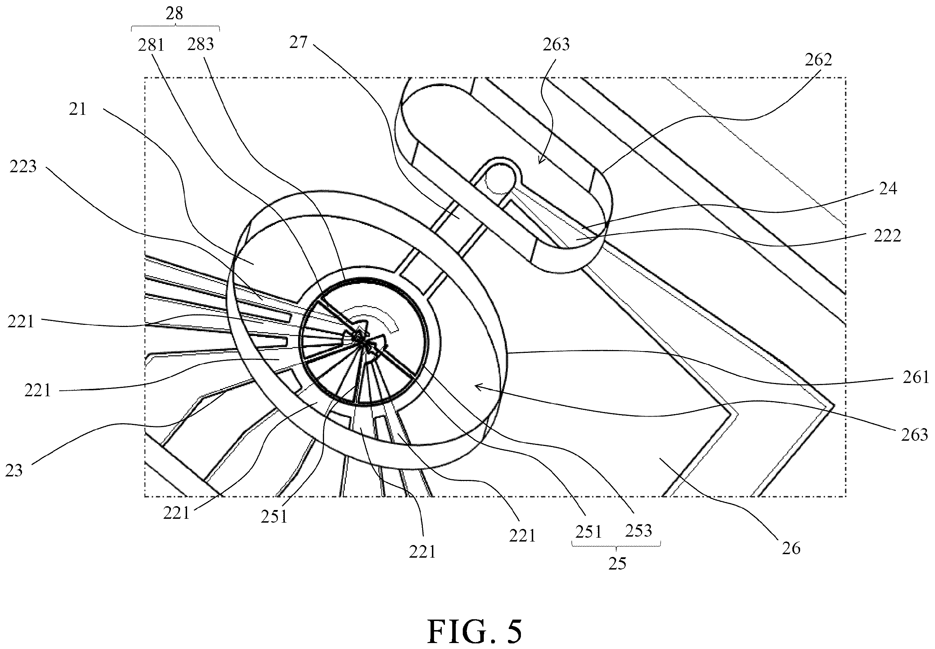

As shown in FIG. 4 and 5, the salt bridge connecting layer 27 is encircled by the insulating layer 23. This is because, in Example 2, during the manufacturing process of the electrochemistry detection chip 20, the insulating layer 23 is not only formed on the electrode chip 22 to cover it, but also further surrounds the area that will be used to form the salt bridge connecting layer 27. Therefore, when a polyelectrolyte is subsequently used to make the salt bridge connecting layer 27 to form a proton-exchange membrane, the insulating layer 23 can be used to confine the polyelectrolyte to the area where the salt bridge connecting layer 27 is to be formed. Thus, the polyelectrolyte can subsequently solidify to form a salt bridge connecting layer 27 having a desire shape and position. However, the present invention is not limited thereto.

Preparation Example:

The electrochemistry detection chips of Example 1 and Example 2 can be manufactured by the process of the Preparation Example, but the present disclosure is not limited thereto.

The electrochemistry detection chip of the Preparation Example is manufactured by microfabrication, comprising: slide cleaning, positive resist coating and developing, platinum (Pt) thin film sputtering, lift-off process, SU8 negative resist circuit insulating layer, and stepped dry negative resist process:

   a. slide cleaning: the base layer is made of slide in Preparation Example, due to the adhesion of metal sputtering will be effected by slide surface cleanliness, first, it needs to clear the oil mark and dust of slide surface by isopropanol (IPA), piranha solution (96% $H_2SO_4$ / 30% $H_2O_2$ ratio 3:1) and distilled water.

   b. Pt electrode manufacturing: using the lift-off process, coating AZ4620 positive resist, and obtaining the designed shape by exposure developing (the shape of Pt electrode is referred to FIG. 1-3 or FIG. 4-6), then, using sputtering method to plat the metal. Wherein, the working electrode (WE) can be a diameter of 20 $\mu$m ultramicroelectrode (UME), its small electrode area causes the low oxygen consumption of electrode itself, and is suitable for assess embryo activity without affecting embryonic respiration and metabolism; the counter electrode (CE) can be used to complete current loop, its area is over 10 times of working electrode, which can provide a role corresponding to the current of working electrode; and the reference electrode (RE) which is located in connecting reference well and controls the electrochemistry reaction rate of WE by providing the reference potential to WE while it is reacting.

   c. manufacturing of insulating layer, first structural layer and second structural layer: To achieve the electro-insulating and form the opening of the electrodes, coating the negative resist SU8-3010 as the insulating layer on Pt electrode of the chip. It can paste a layer of dry negative resist on the insulating layer as the first structural layer to manufacture the first structural layer only electrochemistry detection chip of Example 1. It can paste 2 layers of dry negative resists on the insulating layer as the first and the second structural layers to manufacture the electrochemistry detection chip of Example 2 which has the first and the second structural layers.

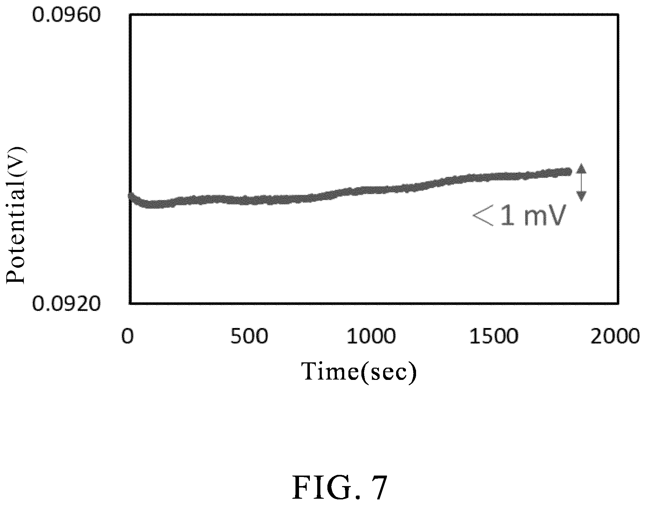

   d. manufacturing of connecting Ag/AgCl reference electrode: coating the mixture of electrical conductive Ag and Ag/Cl on the Pt reference electrode as the reference material layer, and heat over 90°C to dry the mixture. Coating the nafion solution in the salt bridge connecting area between the reference electrode and the working well, and heat over 50 °C to dry the nafion solution. Above these, the connecting Ag/AgCl reference electrode can obtain more stable reference potential, and obtain the more accurate OCR measurement result, the measurement result is referred to FIG. 7. FIG. 7 shows that the connecting Ag/AgCl reference electrode of the electrochemistry detection chip of Example 2 and a commercial Ag/AgCl, which is disposed in working well, can provide a stable reference potential with less than 1 mV amplitude potential of the chipped reference electrode, which is measured by open circuit potential.

   e. manufacturing and design of covering layer microwell, wherein the impermeable material can be poly(dimethylsiloxane) (PDMS) or poly(methylmethacrylate) (PMMA): After finishing the mold by 3D printing, sylgard 184 silicone elastomer and sylgard 184 silicone elastomer curing agent are mixed uniformly by stirring at a weight ratio 10: 1. Use a vacuum pump to remove the bubbles of PDMS mixture liquid which is produced by stirring, and pour the PDMS mixture liquid into master mold, then place on the 75°C heat plate for an hour to make it solidify. After PDMS solidification, remove the PDMS from the master mold and bind the PDMS with the electrochemistry detection chip by $O_2$ plasma treatment. It can also use a laser cutting-off machine to cut the thickness of 5 mm PMMA slab to form a first pore and a second pore, and bind the PMMA slab with the electrochemistry detection chip by impermeable glue to form the reference well and the working well.

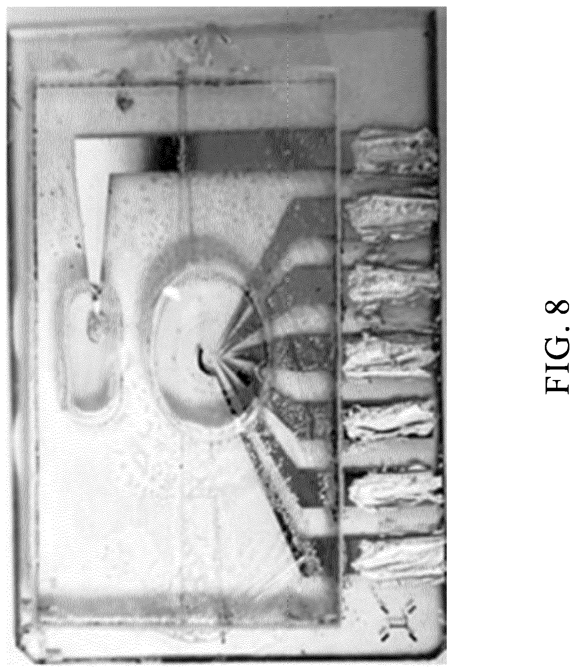

The product image of the electrochemistry detection chip which completes in Example 1 is referred to FIG. 8.

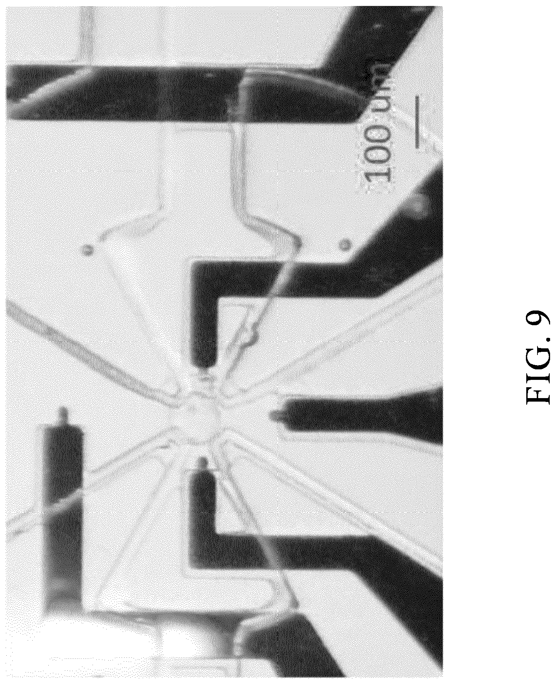

The micrograph of the mice embryo locating on the specimen accommodation area of the electrochemistry detection chip of Example 2 is referred to FIG. 9. Through the stepped structure, which is formed by the first structural layer and the second structural layer, the electrochemistry detection chip can ensure that the mice embryo remains stable in the specimen accommodation area, to ensure that the measurement of oxygen consumption rate will not produce unstable data due to specimen movement.

Test Example:

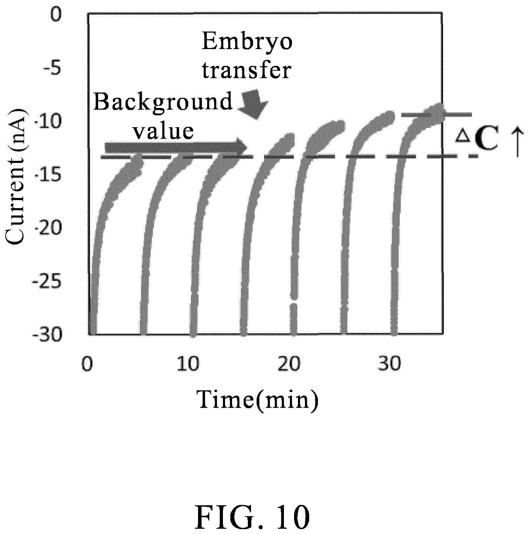

Before placing the specimen on the electrochemistry detection chip of Example 1, drop the NaCl solution into the reference well, and use the cover to avoid its evaporation. After balancing for at least 30 minutes, measure the background current value for 40 seconds by amperometry with dissolved oxygen reduction potential (about -0.65 V) in the culture medium, and wait for 260 seconds. Place the embryo in the specimen accommodation area in the working well, and wait for a minute to measure. Measure for 40 seconds, then pause for 260 seconds, repeat this process 4 times, and calculate by the following formulas, the result is referred to as FIG. 10.

$$\frac{C(r)}{c^*} = \frac{i}{i^*} \ldots\ldots \text{Formula I}$$

$$c(r) = \frac{(c_s - c^*)r_s}{(r + r_s)} + c^* \ , \ \Delta C = C^* - Cs \ldots\ldots \text{Formula II}$$

Wherein r means the distance between electrode and embryo surface, $r_s$ means the radius of embryo, C* means the background solution, and $C_s$ means the oxygen concentration of embryo surface. $C_{(r)}$ means the oxygen concentrations at distances from the embryo surface to different working electrodes (WEs).

Convert the current value into the dissolved oxygen concentration of this electrode distance at 37°C, where the dissolved oxygen concentration is about 165 $\mu$M (Formula I), and estimate the oxygen concentration around the embryo surface by spherical diffusion theory (Formula II). The oxygen consumption rate (OCR) can be expressed as:

$$\text{OCR} = 2\pi r_s D \Delta C \ldots\ldots \text{Formula III}$$

Wherein D means oxygen diffusion coefficient, and it is $2.1 \times 10^3$ $\mu m^2/s$ in 37°C water solution.

From Formula II, it can be seen that the distance between the embryo and the electrode will affect the oxygen concentration calculation of the embryo surface. Therefore, to locate embryo location efficiently and to operate easily are very important. In the present disclosure, it can locate embryo location efficiently, and let the embryologist fix the different developing state embryos in a position offset by about 10 $\mu$m from the center of the structure by the plurality of bottom support arms of the first structural layer (FIG. 9).

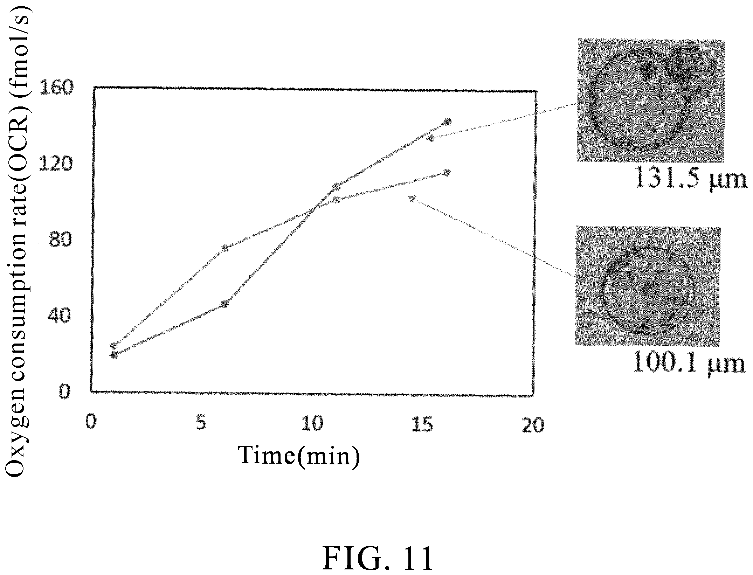

Calculate the oxygen concentration of the embryo surface using Formula III, and assess the respiration activity of the embryo in different diameters. The result is referred to FIG. 11. It further efficiently classifies the embryos with different health levels.

According to the above results, the electrochemistry detection chip of the present disclosure can make the reference electrode to the connecting Ag/AgCl reference electrode by disposition of the reference material layer, and can locate the connecting Ag/AgCl reference electrode in the independent reference well by disposition of the covering layer. These can efficiently reduce the amplitude of the reference potential and obtain more accurate detection results. Besides, the electrochemistry detection chip of the present disclosure can precisely locate the specimen on the specimen accommodation area by disposition of the first structure layer, and avoid the detection results being affected by the specimen movement.

In the preferred embodiment of the present disclosure, further adding the second structural layer can make the specimen's location in the specimen accommodation area easier and more precise due to the wider structure opening of the accommodation area, which can reduce the embryo positioning time from an incubator to the embryo accommodation area and avoid the detection results affected by the specimen movement more efficiently.

While the present disclosure has been described by means of specific embodiments, numerous modifications and variations could be made thereto by those skilled in the art without departing from the scope and spirit of the present disclosure set forth in the claims.

Legends

> 10 electrochemistry detection chip
> 11 base layer
> 12 electrode chip
> 121 working electrode
> 122 reference electrode
> 123 counter electrode
> 13 insulating layer
> 14 reference material layer
> 15 first structural layer
> 151 bottom support arm
> 152 specimen accommodation area
> 16 covering layer
> 161 first pore
> 162 second pore
> 163 working well
> 164 reference well
> 17 salt bridge connecting layer
> 20 electrochemistry detection chip
> 21 base layer
> 22 electrode chip
> 221 working electrode
> 222 reference electrode
> 223 counter electrode
> 23 insulating layer
> 24 reference material layer
> 25 first structural layer
> 251 bottom support arm
> 252 specimen accommodation area
> 253 bottom frame
> 26 covering layer
> 261 first pore
> 262 second pore
> 263 working well
> 264 reference well
> 27 salt bridge connecting layer
> 28 second structural layer
> 281 upper support arm
> 283 upper frame

The legend of the representative drawing is as follows:

> 10 electrochemistry detection chip
> 11 base layer
> 12 electrode chip
> 121 working electrode
> 122 reference electrode
> 123 counter electrode
> 13 insulating layer
> 14 reference material layer
> 15 first structural layer
> 16 covering layer

161 first pore
162 second pore
163 working well
164 reference well
17 salt bridge connecting layer

**Claims**

1. An electrochemistry detection chip (10, 20) which comprises:

   a base layer (11, 21) which is composed of impermeable material;
   an electrode chip (12, 22) which is disposed on the base layer (11, 21), and comprises at least a working electrode (121, 221), a reference electrode (122, 222) and a counter electrode (123, 223);
   an insulating layer (13, 23) which is covered on the electrode chip (12, 22), and make the working electrode (121, 221) exposed at the end only;
   a reference material layer (14, 24) which is disposed on the reference electrode (122, 222) and is composed of silver chloride (AgCl);
   a first structural layer (15, 25) which is disposed on the insulating layer (13, 23) and includes:
   a plurality of bottom support arms (151, 251), wherein the ends of the bottom support arms (151, 251) are adjacent to each other to jointly define a specimen accommodation area (152, 252);
   a covering layer (16, 26) which is disposed on the base layer (11, 21) and is composed of impermeable material, wherein the covering layer (16, 26) has a first pore (161, 261) and a second pore (162, 262), wherein the first pore (161, 261) and a top surface of the base layer (11, 21) jointly define a working well (163, 263), and an end of the working electrode (121, 221) and the counter electrode (123, 223), and the specimen accommodation area (152, 252) are located in the working well (163, 263); wherein the second pore (162, 262) and
   a top surface of the base layer (11, 21) jointly define a reference well (164, 264), and an end of the reference electrode (122, 222) is located in the reference well (164, 264);
   a salt bridge connecting layer (17, 27) which is disposed on the base layer (11, 21), and one end of it is located in the working well (163, 263), and the other end of it is located in the reference well (164, 264).

2. The electrochemistry detection chip (20) according to claim 1 further comprises:
   a second structural layer (28) which is disposed on the first structural layer (25) and comprises:
   a plurality of upper support arms (281), wherein the upper support arms (281) correspond to the settings of the bottom support arms (251) and are adjacent to the specimen accommodation area (252), and the ends of the upper support arms (281) and the bottom support arms (251) form a stepped structure.

3. The electrochemistry detection chip (20) according to claim 2, wherein the end of the upper support arms (281), which are adjacent to the specimen accommodation area (252), can define a specimen accommodation area (252) with a diameter of 100-300 μm.

4. The electrochemistry detection chip (20) according to claim 2, wherein the first structural layer (25) can further comprise:
   a bottom frame (253), wherein the bottom support arms (251) extend inward from the bottom frame (253);
   wherein the second structural layer (28) further comprises:
   an upper frame (283) corresponds to the setting of the bottom frame (253), wherein the upper support arms (281) extend inward from the upper frame(283).

5. The electrochemistry detection chip (10, 20) according to any one of claims 1 to 4, wherein the electrode chip (12, 22) comprises a plurality of working electrodes (121, 221), and the plurality of working electrodes (121, 221) have different distances from the center of the specimen accommodation area (152, 252).

6. The electrochemistry detection chip (10, 20) according to claim 5, wherein the electrode chip (12, 22) comprises 5 working electrodes (121, 221).

7. The electrochemistry detection chip (10, 20) according to claim 5, wherein the specimen accommodation area (152, 252) can be suitable for accommodating a single embryo or a spherical cell cluster.

FIG. 1

FIG. 2

FIG. 3

EP 4 682 529 A1

FIG. 4

FIG. 5

FIG. 6

FIG. 7

FIG. 8

FIG. 9

FIG. 10

EP 4 682 529 A1

131.5 μm

100.1 μm

Oxygen consumption rate(OCR) (fmol/s)

Time(min)

FIG. 11

Europäisches
Patentamt

European
Patent Office

Office européen
des brevets

# EUROPEAN SEARCH REPORT

**Application Number**

EP 25 19 0320

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2009 174948 A (NAT INST ENVIRONMENTAL STUDIES) 6 August 2009 (2009-08-06) <br> * abstract * <br> * figures * <br> * paragraphs [0002], [0008] - [0050] * <br> ----- | 1-7 | INV. <br> G01N27/403 <br> G01N27/416 <br> G01N33/483 <br> G01N33/487 |
| Y | WO 2018/006865 A1 (LEADWAY LTD [CN]) 11 January 2018 (2018-01-11) <br> * abstract * <br> * figures 1,2 * <br> * page 9, third paragraph * <br> ----- | 1-7 | ADD. <br> C12M1/34 |
| Y | JP 2023 522804 A (BOE TECHNOLOGY GROUP CO LTD) 1 June 2023 (2023-06-01) <br> * abstract * <br> * figure 14 * <br> * paragraph [0169] * <br> ----- | 1-7 | |
| Y | US 11 016 078 B2 (JAPAN AVIATION ELECTRONICS IND LTD [JP]; UNIV TOHOKU [JP]) 25 May 2021 (2021-05-25) <br> * abstract * <br> * figures * <br> * column 9, lines 26-32 * <br> ----- | 5,6 | |
| | | | TECHNICAL FIELDS SEARCHED (IPC) <br> C12M <br> G01N |
| A | EP 0 299 778 A2 (STANFORD RES INST INT [US]) 18 January 1989 (1989-01-18) <br> * abstract * <br> * figure 12 * <br> * page 12, lines 37-43 * <br> ----- | 1-7 | |
| A | EP 3 428 632 A1 (PANASONIC IP MAN CO LTD [JP]) 16 January 2019 (2019-01-16) <br> * abstract * <br> * figures * <br> ----- <br> -/-- | 1-7 | |

The present search report has been drawn up for all claims

| Place of search | Date of completion of the search | Examiner |
|---|---|---|
| Munich | 28 October 2025 | Ruchaud, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 25 19 0320

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 113 702 468 A (WUXI JINBOCHENG MEDICAL APPARATUS TECH CO LTD) 26 November 2021 (2021-11-26) * abstract * * figure 1 * * paragraph [0026] * ----- | 1-7 | |
| A | US 2021/278346 A1 (WU FUH-TSANG [TW] ET AL) 9 September 2021 (2021-09-09) * abstract * * figure 1 * * paragraph [0053] * ----- | 1-7 | |
| | | | **TECHNICAL FIELDS SEARCHED (IPC)** |

The present search report has been drawn up for all claims

| Place of search | Date of completion of the search | Examiner |
|---|---|---|
| Munich | 28 October 2025 | Ruchaud, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................................................
& : member of the same patent family, corresponding document

EPO FORM 1503 03.82 (P04C01)

page 2 of 2

# EP 4 682 529 A1

## ANNEX TO THE EUROPEAN SEARCH REPORT
## ON EUROPEAN PATENT APPLICATION NO.

EP 25 19 0320

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2025

| Patent document cited in search report | | | Publication date | Patent family member(s) | | Publication date |
|---|---|---|---|---|---|---|
| JP 2009174948 | A | | 06-08-2009 | NONE | | |
| WO 2018006865 | A1 | | 11-01-2018 | CN | 106198950 A | 07-12-2016 |
| | | | | CN | 113466443 A | 01-10-2021 |
| | | | | WO | 2018006865 A1 | 11-01-2018 |
| JP 2023522804 | A | | 01-06-2023 | CN | 112834763 A | 25-05-2021 |
| | | | | CN | 211905402 U | 10-11-2020 |
| | | | | EP | 4067907 A1 | 05-10-2022 |
| | | | | JP | 2023522804 A | 01-06-2023 |
| | | | | KR | 20230005803 A | 10-01-2023 |
| | | | | US | 2022241774 A1 | 04-08-2022 |
| | | | | WO | 2021218537 A1 | 04-11-2021 |
| US 11016078 | B2 | | 25-05-2021 | CN | 108291891 A | 17-07-2018 |
| | | | | CN | 111505090 A | 07-08-2020 |
| | | | | EP | 3379241 A1 | 26-09-2018 |
| | | | | EP | 4212863 A1 | 19-07-2023 |
| | | | | JP | 6116075 B1 | 19-04-2017 |
| | | | | JP | 2017096721 A | 01-06-2017 |
| | | | | SG | 11201804099R A | 28-06-2018 |
| | | | | TW | 201719163 A | 01-06-2017 |
| | | | | US | 2020173978 A1 | 04-06-2020 |
| | | | | US | 2021247376 A1 | 12-08-2021 |
| | | | | WO | 2017086059 A1 | 26-05-2017 |
| EP 0299778 | A2 | | 18-01-1989 | CA | 1298873 C | 14-04-1992 |
| | | | | EP | 0299778 A2 | 18-01-1989 |
| | | | | JP | H01112149 A | 28-04-1989 |
| | | | | US | 4874500 A | 17-10-1989 |
| EP 3428632 | A1 | | 16-01-2019 | CN | 108603857 A | 28-09-2018 |
| | | | | EP | 3428632 A1 | 16-01-2019 |
| | | | | JP | 6492294 B2 | 03-04-2019 |
| | | | | JP | WO2017154801 A1 | 10-01-2019 |
| | | | | US | 2018364190 A1 | 20-12-2018 |
| | | | | WO | 2017154801 A1 | 14-09-2017 |
| CN 113702468 | A | | 26-11-2021 | NONE | | |
| US 2021278346 | A1 | | 09-09-2021 | TW | 202134646 A | 16-09-2021 |
| | | | | US | 2021278346 A1 | 09-09-2021 |

EPO FORM P0459

For more details about this annex : see Official Journal of the European Patent Office, No. 12/82

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010121948 A
- US 10458941 B2
- CN 107003265 A
- CN 117030824 A
- TW 201120441 A1